# EUROPEAN PATENT APPLICATION

(11) **EP 4 692 788 A1**
(43) Date of publication of application: **11.02.2026**
(21) Application number: 24218685.6
(22) Date of filing: 10.12.2024
(51) Int. Cl.: G01N 33/00, G01N 1/38

(54) **DEVICE FOR MEASURING ODOR**

(30) Priority: 06.08.2024 KR 20240104521
(71) Applicant: Hyundai Motor Company, Seoul 06797 (KR); Kia Corporation, Seocho-gu Seoul 06797 (KR)
(72) Inventor: LEE, Tae Hee, 18280 Hwaseong-si, Gyeonggi-do (KR); SUNG, Dae Un, 18280 Hwaseong-si, Gyeonggi-do (KR); LEE, Kyung Woo, 18280 Hwaseong-si, Gyeonggi-do (KR)
(74) Representative: Viering, Jentschura & Partner mbB Patent- und Rechtsanwälte

(57) **Abstract**

A device for measuring odor has a pre-chamber having a gas flow chamber therein in which the gas of an odor measurement target or fresh air flows. The device additionally has a vortex generation block, a gas distribution guide, or the like in the gas flow chamber, thereby being able to prevent mixing of the gas of the odor measurement target and the fresh air that flow through the gas flow chamber. The device is able to prevent errors in measurement data that is measured by an odor sensor and provides the accuracy of odor data by enabling the gas of the odor measurement target to be uniformly mixed and distributed and then supplied to the odor sensor such that the gas can be sensed.

## Description

### BACKGROUND

### Technical Field

The present disclosure relates to a device for measuring odor and, in more detail, a device for measuring odor that prevents mixing of a gas of an odor measurement target and fresh air and uniformly distributes the gas of the odor measurement target such that the gas can be supplied to an odor sensor.

### Description of the Related Art

The peculiar odors of various interior materials of a vehicle such as the seat covers, head lining, door trims, mats, the odors of volatile organic compounds (VOC), and the like are factors of main complaints about new vehicles.

Further, propagation of mold and bad smells that are generated by moisture condensing in an evaporator when the air conditioning system of a vehicle is operated are also causes of discomfort for users of vehicles.

Various bad smells may be contained in external air that flows into a vehicle from the outside when the vehicle is driven, depending on the surroundings, and bad smells flowing inside from the outside may cause discomfort to users of vehicles.

As described above, various odors generated inside and outside vehicles may degrade the emotional quality users' experiences of vehicles.

Accordingly, accurate measurement through an odor sensor and establishment of odor data must precede for analysis of the causes of various odors that are generated inside and outside vehicles and for measures for removing the odors. There is a need for a process of clearly determining the substantial causes of bad smells by analyzing the components and concentrations of various odors that are generated inside and outside vehicles.

To this end, a process of accurately measuring odor data including the components, concentrations, and the like of odor by installing odor sensors at relevant industrial sites must be preceded to solve civil petitions related to bad smells not only inside and outside a vehicle, but at various industrial sites.

In this case, electric-chemical odor sensors and electric-chemical odor sensor arrays may be used for the odor sensors, or bio odor sensors such as a bio peptide-type sensor and a sensor using amino acid may be used. Odor data measured through such odor sensors can be obtained for a predetermined time.

However, there is a problem that the gas of an odor measurement target is supplied to an odor sensor without uniformly spreading over a predetermined time or the gas of an odor measurement target and fresh air are mixed and supplied to an odor sensor, so the measurement accuracy of odor sensors is deteriorated. For example, the odor concentration measured by an odor sensor may be lower than the actual odor concentration.

Further, odor data that is measured by an odor sensor may depend on the temperature and humidity of the gas of an odor measurement target, and accordingly, there is a problem that the measurement accuracy of an odor sensor is deteriorated.

### SUMMARY

The present disclosure has been made in an effort to solve the problems of the related art described above. An objective of the present disclosure is to provide a device for measuring odor that has a pre-chamber having a gas flow chamber therein in which the gas of an odor measurement target or fresh air flows and having a vortex generation block, a gas distribution guide, and the like in the gas flow chamber, thereby being able to prevent mixing of the gas of an odor measurement target and fresh air that flow through the gas flow chamber. A further objective of the present disclosure is being able to prevent errors in measurement data that is measured by an odor sensor and to provide the accuracy of odor data by enabling the gas of an odor measurement target to be uniformly mixed and distributed and then supplied to the odor sensor such that the gas can be sensed.

In order to achieve the objectives, the present disclosure provides a device for measuring odor, the device including a pre-chamber having a gas flow chamber formed with a predetermined depth on a top thereof in which a gas of an odor measurement target or fresh air flows, and having a gas distribution guide formed at a predetermined position on a bottom of the gas flow chamber to distribute the gas of the odor measurement target or the fresh air. The present disclosure further includes a first fresh air supply pipe and a second fresh air supply pipe connected to respective sides on a front of the pre-chamber to supply the fresh air to the gas flow chamber. The present disclosure further includes a gas supply pipe connected at a position between the first fresh air supply pipe and the second fresh air supply pipe on the front of the pre-chamber to supply the gas of the odor measurement target to the gas flow chamber. The present disclosure further includes a gas discharge pipe connected to a rear of the pre-chamber to discharge the fresh air or the gas of the odor measurement target to an odor sensor.

The device for measuring odor according to the present disclosure may further include: an odor sensor chamber connected to the gas discharge pipe; and the odor sensor disposed in the odor sensor chamber and configured to measure odor of the gas of the odor measurement target.

A gas intake hole connected with the gas supply pipe may be formed at a middle position in a width direction on a front wall of the gas flow chamber. A first air intake hole connected with the first fresh air supply pipe and a second air intake hole connected with the second fresh air supply pipe may be formed at respective sides in a width direction on a front wall of the gas flow chamber, respectively.

A gas discharge hole connected with the gas discharge pipe may be formed on a rear wall of the gas flow chamber.

The gas flow chamber may be composed of a first gas flow chamber having a shape gradually narrowing in a gas flow direction and a second gas flow chamber having a straight shape having a constant width.

The gas distribution guide may be formed on a bottom of the second gas flow chamber, may be formed in a bar shape shorter in length than the second gas flow chamber, and may be disposed in a longitudinal direction of the second gas flow chamber at a middle position in a width direction of the second gas flow chamber.

A vortex generation block configured to generate a vortex of the gas of the odor measurement target may be formed at a predetermined distance forward from a front end of the gas distribution guide on a bottom of the gas flow chamber.

The vortex generation block may be formed in a semicircular structure being convex toward a front of the gas flow chamber in the opposite direction to a flow direction of the gas of the odor measurement target.

A plurality of prevention blocks configured to prevent contamination gas from accumulating may be formed at both sides of the first gas flow chamber of the gas flow chamber.

A heater configured to heat gas flowing in the gas flow chamber and a cooler configured to cool gas flowing in the gas flow chamber may be mounted on an outer surface of the pre-chamber.

The present disclosure provides the following effects from the objectives described above.

Since the gas of the odor measurement target passing through the gas flow chamber of the pre-chamber is uniformly distributed and mixed by the vortex generation block and the gas distribution guide and then can be supplied to the odor sensor such that the gas can be sensed, it is possible to prevent errors in measurement data that is measured by an odor sensor and provides accuracy of the odor data.

Since contamination gas remaining in the gas flow chamber (the gas of a previous odor measurement target) can be easily removed by supplying fresh air into the gas flow chamber of the pre-chamber after the gas of the odor measurement target is measured, it is possible to prevent the gas of a next odor measurement target that is supplied into the gas flow chamber of the pre-chamber from mixing with the contamination gas (the gas of the previous odor measurement target).

Since the plurality of prevention blocks are further formed in the gas flow chamber of the pre-chamber, it is possible to prevent contamination gas (the gas of a previous odor measurement target) from intensively remaining at both sides in the gas flow chamber of the pre-chamber after odor is measured by the odor sensor.

Fresh air is pushed out of the gas flow chamber when the gas flow chamber of the pre-chamber is filled with the gas of the odor measurement target, and the gas of the odor measurement target is pushed out of the gas flow chamber when the gas flow chamber of the pre-chamber is filled with fresh air, thereby preventing mixing of the gas of the odor measurement target and the fresh air. Accordingly, it is possible to limit the measurement target of the odor sensor to only the gas of the odor measurement target, whereby it is possible to improve the accuracy in measurement of the odor sensor.

The temperature and humidity of the gas of the odor measurement target can be adjusted at predetermined levels by the heater and the cooler mounted on the pre-chamber, and accordingly, it is possible to provides accuracy of odor data that is measured by the odor sensor.

### BRIEF DESCRIPTION OF THE DRAWINGS

FIG. 1 and FIG. 2 are perspective views showing a pre-chamber of a device for measuring odor according to an embodiment of the present disclosure.
FIG. 3 is a plan view showing the device for measuring odor including the pre-chamber according to an embodiment of the present disclosure.
FIG. 4 is a plan view showing the state in which the gas of an odor measurement target is distributed in the pre-chamber of the device for measuring odor according to an embodiment of the present disclosure.
FIG. 5 is a plan view showing the state in which fresh air is supplied to the pre-chamber of the device for measuring odor according to an embodiment of the present disclosure.
FIG. 6 is a plan view showing a device for measuring odor including a pre-chamber according to another embodiment of the present disclosure.
FIG. 7 is a plan view showing the state in which the gas of an odor measurement target is distributed in the pre-chamber of the device for measuring odor according to another embodiment of the present disclosure.
FIG. 8 is a plan view showing the state in which fresh air is supplied to the pre-chamber of the device for measuring odor according to another embodiment of the present disclosure.
FIG. 9 is a vertical cross-sectional view showing an example when a pre-chamber of a device for measuring odor according to the present disclosure is equipped with a heater and a cooler.

### DETAILED DESCRIPTION

Descriptions of specific structures and functions disclosed in embodiments of the present disclosure are only examples for describing the embodiments according to the concept of the present disclosure and the embodiments according to the concept of the present disclosure may be implemented in various ways. The present disclosure is not limited to the embodiments described herein and should be construed as including all changes, equivalents, and replacements that are included in the spirit and the range of the present disclosure.

It is understood that, although the terms first and/or second, and the like may be used herein to describe various elements, these elements should not be limited by these terms. These terms are used only to distinguish one element from another element. For instance, a first element discussed below could be termed a second element without departing from the right range of the present disclosure. Similarly, the second element could also be termed the first element.

It is to be understood in the specification that when one element is referred to as being "connected to" or "coupled to" another element, it may be connected directly to or coupled directly to another element or be connected to or coupled to another element, having some other element intervening therebetween. On the other hand, it is to be understood that when one element is referred to as being "connected directly to" or "coupled directly to" another element, it may be connected to or coupled to that other element without another element intervening therebetween. Further, the terms used herein to describe a relationship between elements, i.e., "between", "directly between", "adjacent", or "directly adjacent" should be interpreted in the same manner as those described above.

Like reference numerals indicate the same components throughout the specification. The terms used herein are provided to describe embodiments without limiting the present disclosure. In the specification, a singular form includes a plural form unless specifically stated in the sentences. The terms "comprise" and/or "comprising" used herein do not exclude that another component, step, operation, and/or element exist or are added in the stated component, step, operation, and/or element.

When a component, device, element, or the like of the present disclosure is described as having a purpose or performing an operation, function, or the like, the component, device, or element should be considered herein as being "configured to" meet that purpose or to perform that operation or function.

Hereinafter, embodiments of the present disclosure are described in detail with reference to the accompanying drawings.

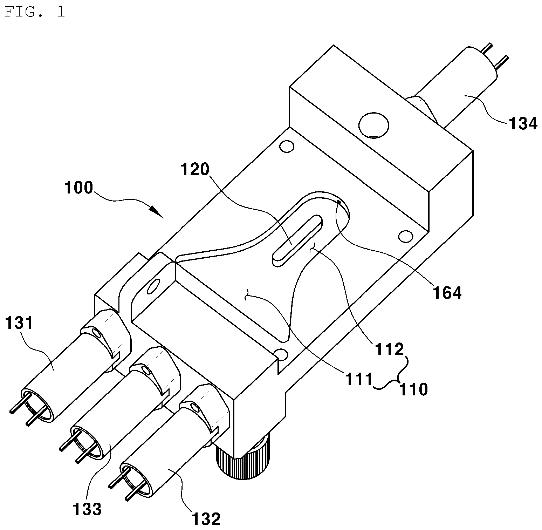

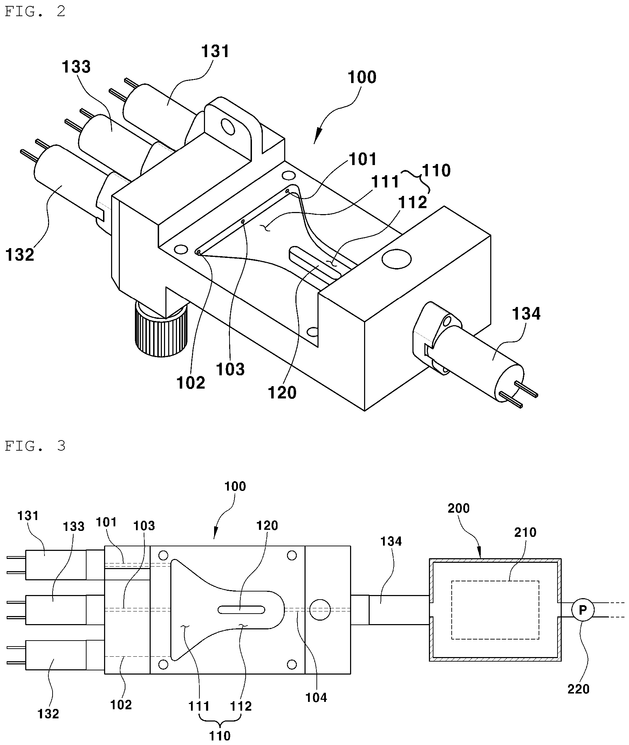

In the accompanying drawings, FIG. 1 and FIG. 2 are perspective views showing a pre-chamber of a device for measuring odor according to an embodiment of the present disclosure. FIG. 3 is a plan view showing the device for measuring odor including the pre-chamber according to an embodiment of the present disclosure.

The device for measuring odor includes a pre-chamber 100 and an odor sensor chamber 200 having an odor sensor 210 installed therein.

The pre-chamber 100 has a structure that can uniformly distribute and then supply the gas of an odor measurement target (i.e., an odorous gas) or fresh air to the odor sensor chamber 200.

To this end, a gas flow chamber 110 through which the gas of an odor measurement target or fresh air flows is formed with a predetermined depth on the top of the pre-chamber 100. A gas distribution guide 120 for uniformly distributing the gas of an odor measurement target or fresh air is formed with a predetermined height at a predetermined position on the bottom of the gas flow chamber 110.

The top of the gas flow chamber 110 of the pre-chamber 100 is open in FIG. 1, FIG. 2, and FIG. 3, but a separate sealing block (not shown) for sealing the open top of the gas flow chamber 110 may be fastened to the top of the pre-chamber 100.

The gas flow chamber 110 may be divided into a first gas flow chamber 111 and a second gas flow chamber 112 connected to each other.

In detail, the gas flow chamber 110 may be divided into a first gas flow chamber 111 having a channel shape gradually narrowing in the gas flow direction and a second gas flow chamber 112 having a straight channel shape having a constant width.

In this configuration, the reason that the first gas flow chamber 111 of the gas flow chamber 110 is formed in a gradually narrowing channel shape is, as described below, for allowing a gas intake hole 103 through which the gas of an odor measurement target flows inside and a first air intake hole 101 and a second air intake hole 102 through which fresh air flows inside being formed at different positions on the front wall of the gas flow chamber 110 and for intensively guiding the gas of an odor measurement target or fresh air to the second gas flow chamber 112.

The gas distribution guide 120 may be formed on the bottom of the second gas flow chamber 112 while having a height the same as the depth of the gas flow chamber 110 and serves to uniformly distribute and mix the gas of an odor measurement target or fresh air flowing from the first gas flow chamber 111 and then discharge the mixture to the odor sensor chamber 200.

To this end, the gas distribution guide 120 is formed in a bar shape shorter than the second gas flow chamber 112 and is disposed in the longitudinal direction of the second gas flow chamber 112 at a middle position in the width direction of the second gas flow chamber 112.

A first fresh air supply pipe 131 and a second fresh air supply pipe 132 for supplying fresh air to the gas flow chamber 110 are connected to both sides on the front of the pre-chamber 100, respectively.

A gas supply pipe 133 for supplying the gas of an odor measurement target to the gas flow chamber 110 is connected at the middle position in the width direction, i.e., at a position between the first fresh air supply pipe 131 and the second fresh air supply pipe 132 on the front of the pre-chamber 100.

A gas discharge pipe 134 for discharging the fresh air in the gas flow chamber 110 or the gas of an odor measurement target into the odor sensor chamber 200 having the odor sensor 210 therein is connected to the rear of the pre-chamber 100.

The gas intake hole 103 is connected with the gas supply pipe 133 at a middle position in the width direction on the front wall of the gas flow chamber 110 at the front of the pre-chamber 100. The first air intake hole 101 is connected with the first fresh air supply pipe 131 and the second air intake hole 102 is connected with the second fresh air supply pipe 132 at both sides in the width direction on the front wall of the gas flow chamber 110 at the front of the pre-chamber 100.

A gas discharge hole 104 connected with the gas discharge pipe 134 is formed on the rear wall of the gas flow chamber 110 at the rear of the pre-chamber100.

The odor sensor chamber 200 having a predetermined volume is connected to the gas discharge pipe 134. The odor sensor 210 measuring odor from the gas of an odor measurement target is attached to the bottom inside of the odor sensor chamber 200, and a suction pump 220 is connected to an outlet formed on a second side of the odor sensor chamber 200.

Accordingly, the gas of an odor measurement target or fresh air can be suctioned into the gas flow chamber 110 of the pre-chamber 100 by the suction pump 220 and then can easily flow into the odor sensor chamber 200 through the gas discharge pipe 134 and can be discharged outside through the outlet of the odor sensor chamber 200 after the odor sensor 210 senses odor.

The process of measuring odor by the device for measuring odor according to an embodiment of the present disclosure is described hereafter.

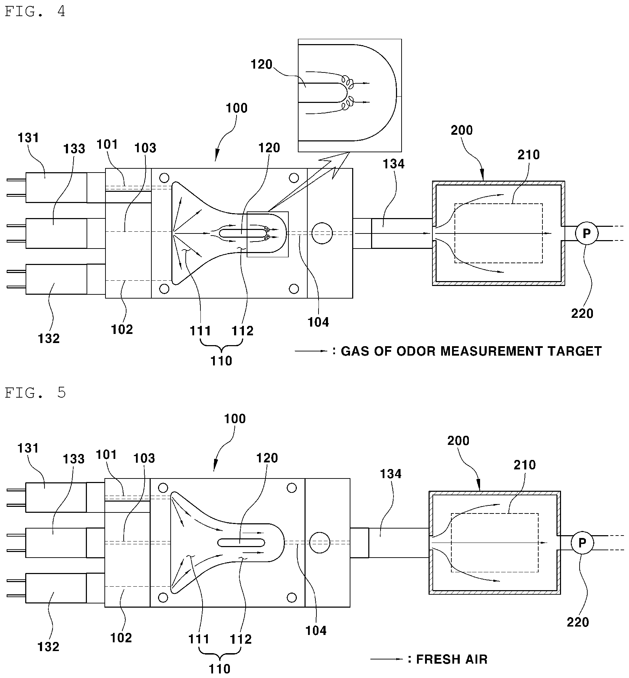

In the accompanying drawings, FIG. 4 is a plan view showing the state in which the gas of an odor measurement target is distributed in the pre-chamber of the device for measuring odor according to an embodiment of the present disclosure. FIG. 5 is a plan view showing the state in which fresh air is supplied to the pre-chamber of the device for measuring odor according to an embodiment of the present disclosure.

First, the gas of a certain kind of odor measurement target is suctioned into the gas flow chamber 110 of the pre-chamber 100 with the suction pump 220 in operation.

In other words, the gas of a certain kind of odor measurement target is suctioned into the first gas flow chamber 111 of the gas flow chamber 110, as shown in FIG. 4, through the gas supply pipe 133 and the gas intake hole 103 with the suction pump 220 in operation.

Next, the gas of the odor measurement target flowing in the first gas flow chamber 111 flows toward the second gas flow chamber 112.

Further, the gas of the odor measurement target flowing toward the second gas flow chamber 112, as shown in FIG. 4, flows through the second gas flow chamber 112 while being distributed into two streams on the front end of the gas distribution guide 120.

When the gas of the odor measurement target distributed by the gas distribution guide 120 flows through the second gas flow chamber 112, the gas generates vortexes at the rear end of the gas distribution guide 120, as shown in FIG. 4 (see inset), whereby the gas can be uniformly mixed.

Accordingly, the gas of the odor measurement target distributed and uniformly mixed by the gas distribution guide 120 can flow to the gas discharge pipe 134 through the gas discharge hole 104 and then can be supplied into the odor sensor chamber 200.

Therefore, since the gas of the odor measurement target distributed and uniformly mixed by the gas distribution guide 120 is easily supplied into the odor sensor chamber 200, the odor of the gas of the odor measurement target can be accurately measured by the odor sensor 210 attached in the odor sensor chamber 200.

Fresh air injected in advance remains in the gas flow chamber 110 of the pre-chamber 100 to remove contamination gas (e.g., the gas of the odor measurement target injected before and remaining). The remaining fresh air can be discharged through the gas discharge pipe 134 and the outlet of the odor sensor chamber 200 by the pressure of the gas of the odor measurement target that is suctioned into the gas flow chamber 110, as described above.

As described above, when the odor of the gas of the odor measurement target is measured by the odor sensor 210, as shown in FIG. 4, fresh air is supplied into the gas flow chamber 110 of the pre-chamber 100, as shown in FIG. 5, whereby the gas of the odor measurement target remaining in the gas flow chamber 110 and the odor sensor chamber 200 can be removed. The odor of the gas of a new odor measurement target can be measured in the order described above.

To this end, fresh air is supplied into the gas flow chamber 110 through the first fresh air supply pipe 131 and the first air intake hole 101 from a fresh air supplier/source. Simultaneously, fresh air is supplied into the gas flow chamber 110 through the second fresh air supply pipe 132 and the second air intake hole 102. Then the fresh air is supplied to the odor sensor chamber 200 through the gas discharge pipe 134, whereby the gas of the previous odor measurement target remaining in the gas flow chamber 110 and the odor sensor chamber 200 can be pushed and removed outside through the outlet of the odor sensor chamber 200. The odor of the gas of a new odor measurement target can be measured in the order described above.

The configuration of a device for measuring odor according to another embodiment of the present disclosure is described hereafter.

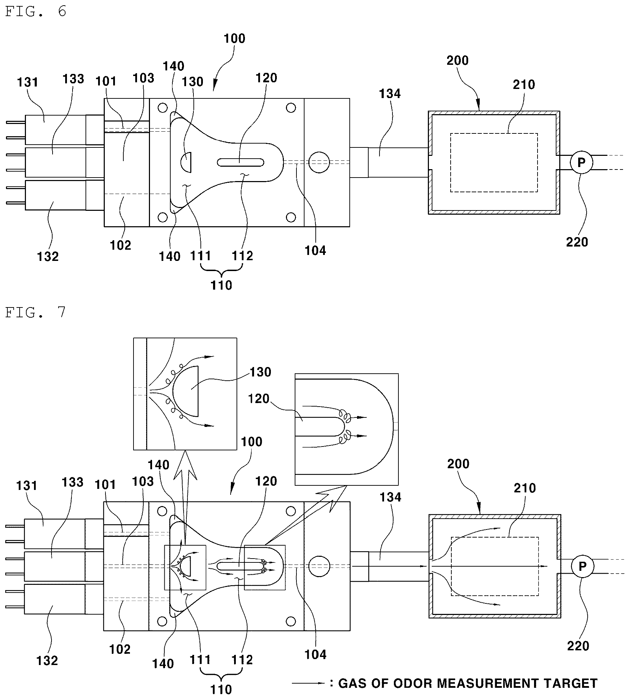

In the accompanying drawings, FIG. 6 is a plan view showing a device for measuring odor including a pre-chamber according to another embodiment of the present disclosure.

The device for measuring odor according to another embodiment of the present disclosure has the same configuration as the embodiment described above and is characterized in that a vortex generation block 130 and a plurality of prevention blocks 140 are further formed in the gas flow chamber 110.

The vortex generation block 130 is formed at a predetermined distance forward from the front end of the gas distribution guide 120 on the bottom of the first gas flow chamber 111 of the gas flow chamber 110 to primarily generate a vortex of the gas of an odor measurement target.

In detail, the vortex generation block 130 is disposed between the gas intake hole 103 and the gas distribution guide 120 to primarily generate a vortex of the gas of an odor measurement target.

The vortex generation block 130 is formed in a semicircular structure having the same height as the gas distribution guide 120 and being convex toward the front of the gas flow chamber 110 in the opposite direction to the flow direction of the gas of an odor measurement target.

The plurality of prevention blocks 140 are formed in a structure that can fill both corners of the first gas flow chamber 111 of the gas flow chamber 110, thereby serving to prevent contamination gas (e.g., the gas of an odor measurement target injected before and remaining) from accumulating or remaining at both corners of the first gas flow chamber 111.

The process of measuring odor by the device for measuring odor according to another embodiment of the present disclosure is described hereafter.

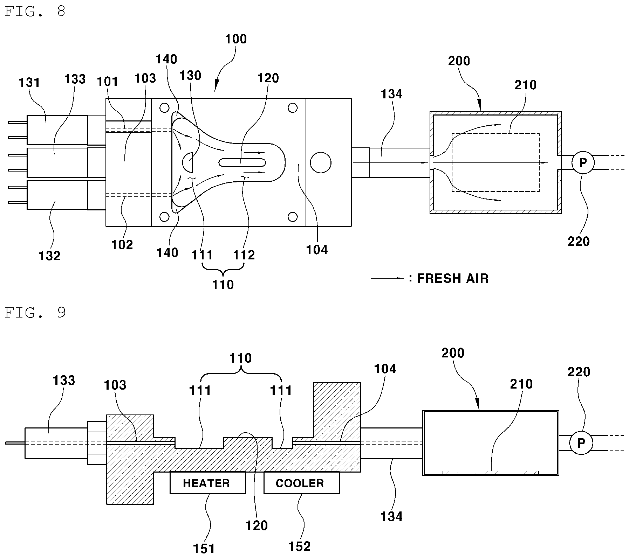

In the accompanying drawings, FIG. 7 is a plan view showing the state in which the gas of an odor measurement target is distributed in the pre-chamber of the device for measuring odor according to another embodiment of the present disclosure. FIG. 8 is a plan view showing the state in which fresh air is supplied to the pre-chamber of the device for measuring odor according to another embodiment of the present disclosure.

First, the gas of a certain kind of odor measurement target is suctioned into the first gas flow chamber 111 of the gas flow chamber 110 through the gas supply pipe 133 and the gas intake hole 103 with the suction pump 220 in operation.

Next, the gas of the odor measurement target flowing in the first gas flow chamber 111 is, as shown in FIG. 7, distributed and mixed while primarily generating a vortex on the front of the vortex generation block 130 and then flows toward the second gas flow chamber 112.

Next, the gas of the odor measurement target flowing toward the second gas flow chamber 112, as shown in FIG. 7, flows through the second gas flow chamber 112 while being distributed into two streams on the front end of the gas distribution guide 120.

When the gas of the odor measurement target distributed by the gas distribution guide 120 flows through the second gas flow chamber 112, the gas secondarily generates vortexes at the rear end of the gas distribution guide 120, as shown in FIG. 7, whereby the gas can be uniformly mixed.

Accordingly, the gas of the odor measurement target distributed and uniformly mixed by the vortex generation block 130 and the gas distribution guide 120 can flow to the gas discharge pipe 134 through the gas discharge hole 104 and then can be supplied into the odor sensor chamber 200.

Therefore, since the gas of the odor measurement target distributed and uniformly mixed by the vortex generation block 130 and the gas distribution guide 120 is easily supplied into the odor sensor chamber 200, the odor of the gas of the odor measurement target can be more accurately measured by the odor sensor 210 attached in the odor sensor chamber 200.

As described above, when the odor of the gas of an odor measurement target is measured by the odor sensor 210, as shown in FIG. 7, fresh air is supplied into the gas flow chamber 110 of the pre-chamber 100, as shown in FIG. 8, whereby the gas of an odor measurement target remaining in the gas flow chamber 110 and the odor sensor chamber 200 can be removed. The odor of the gas of a new odor measurement target can be measured in the order described above.

When the odor of the gas of an odor measurement target flowing in the odor sensor chamber 200 is measured by the odor sensor 210, odor data that is measured by the odor sensor may be changed, depending on the temperature and humidity of the gas, and as a result, the accuracy in measurement of the odor sensor may be deteriorated. Accordingly, it is advantageous to adjust the temperature and humidity of gas that is sensed by the odor sensor 210 to predetermined levels.

To this end, the pre-chamber 100, as shown in FIG. 9, may be equipped with a heater 151 that is controlled to turn on/off by a controller (not shown) to heat the gas passing through the gas flow chamber 110 and a cooler 152 that is controlled to turn on/off by the controller to cool the gas passing through the gas flow chamber 110.

Accordingly, when the temperature of the gas passing through the gas flow chamber 110 is less than a reference level or the humidity of the gas is over a reference level, the controller turns on the heater to operate heating based on a detection signal by a temperature/humidity sensor (not shown). Further, when the temperature of the gas passing through the gas flow chamber 110 is over the reference level or the humidity of the gas is less than the reference level, the controller turns on the cooler 152 to operate for cooling. Accordingly, the temperature and the humidity of the gas or air passing through the gas flow chamber 110 of the pre-chamber 100 can be adjusted at predetermined levels.

Although the present disclosure was described above in detail through various embodiments, the scope of the present disclosure is not limited to the embodiments, and various changes and modifications by those having ordinary skill in the art using the spirit of the present disclosure defined in the following claims are also included in the scope of the present disclosure.

For example, further embodiments of the invention are as follows:

According to a further embodiment of the invention, there is provided a device for measuring odor, the device comprising: a pre-chamber having a gas flow chamber formed on a top thereof in which a gas of an odor measurement target or fresh air flows, and having a gas distribution guide formed in the gas flow chamber to distribute the gas of the odor measurement target or the fresh air; a fresh air supply pipe connected to a front of the pre-chamber to supply fresh air to the gas flow chamber; a gas supply pipe connected to the front of the pre-chamber to supply the gas of the odor measurement target to the gas flow chamber; and a gas discharge pipe connected to a rear of the pre-chamber to discharge the fresh air or the gas of the odor measurement target to an odor sensor, wherein the gas flow chamber is formed wider at the gas supply pipe than at the gas discharge pipe, and the gas distribution guide is formed on a bottom of the gas flow chamber.

## Claims

1. A device for measuring odor, the device comprising:
a pre-chamber having a gas flow chamber formed on a top thereof in which a gas of an odor measurement target or fresh air flows, and having a gas distribution guide formed in the gas flow chamber to distribute the gas of the odor measurement target or the fresh air;
a first fresh air supply pipe and a second fresh air supply pipe connected to respective sides on a front of the pre-chamber to supply the fresh air to the gas flow chamber;
a gas supply pipe connected at a position between the first fresh air supply pipe and the second fresh air supply pipe on the front of the pre-chamber to supply the gas of the odor measurement target to the gas flow chamber; and
a gas discharge pipe connected to a rear of the pre-chamber to discharge the fresh air or the gas of the odor measurement target to an odor sensor.

2. The device of claim 1, further comprising:
an odor sensor chamber connected to the gas discharge pipe; and
the odor sensor disposed in the odor sensor chamber and configured to measure odor of the gas of the odor measurement target.

3. The device of claim 1 or 2, wherein a gas intake hole connected with the gas supply pipe is formed at a middle position in a width direction on a front wall of the gas flow chamber.

4. The device of anyone of claims 1-3, wherein a first air intake hole connected with the first fresh air supply pipe and a second air intake hole connected with the second fresh air supply pipe are formed at respective sides in a width direction on a front wall of the gas flow chamber, respectively.

5. The device of anyone of claims 1-4, wherein a gas discharge hole connected with the gas discharge pipe is formed on a rear wall of the gas flow chamber.

6. The device of anyone of claims 1-5, wherein the gas flow chamber is composed of a first gas flow chamber having a shape gradually narrowing in a gas flow direction and a second gas flow chamber having a straight shape having a constant width.

7. The device of claim 6, wherein the gas distribution guide is formed on a bottom of the second gas flow chamber.

8. The device of claim 6 or 7, wherein the gas distribution guide is formed in a bar shape shorter in length than the second gas flow chamber and is disposed in a longitudinal direction of the second gas flow chamber at a middle position in a width direction of the second gas flow chamber.

9. The device of anyone of claims 1-8, wherein a vortex generation block configured to generate a vortex of the gas of the odor measurement target is formed at a predetermined distance from a front end of the gas distribution guide on a bottom of the gas flow chamber.

10. The device of claim 9, wherein the vortex generation block is formed in a semicircular structure being convex toward a front of the gas flow chamber in the opposite direction to a flow direction of the gas of the odor measurement target.

11. The device of anyone of claims 6-8 or of anyone of claims 9-10 provided that in combination with claim 6, wherein a plurality of prevention blocks configured to prevent contamination gas from accumulating are formed at both sides of the first gas flow chamber of the gas flow chamber.

12. The device of anyone of claims 1-11, wherein a heater configured to heat gas flowing in the gas flow chamber and a cooler configured to cool gas flowing in the gas flow chamber are mounted on an outer surface of the pre-chamber.
